# EUROPEAN PATENT APPLICATION

(11) **EP 4 691 394 A1**
(43) Date of publication of application: **11.02.2026**
(21) Application number: 24780417.2
(22) Date of filing: 27.03.2024
(51) Int. Cl.: A61B 17/70, A61F 2/44, A61L 31/04, A61L 31/12

(54) **ROD FOR INTRASPINAL FIXATION INSTRUMENT IN WHICH BROKEN FRAGMENTS DO NOT READILY SCATTER**

(30) Priority: 31.03.2023 JP 2023058577
(71) Applicant: Globeride, Inc., Higashikurume-shi, Tokyo 203-8511 (JP); Spine-Tech Inc., Tokyo 105-0003 (JP)
(72) Inventor: OIKAWA Katsuhiro, Higashikurume-shi Tokyo 203-8511 (JP); KUSUMOTO Harunobu, Higashikurume-shi Tokyo 203-8511 (JP); MORITA Kohei, Tokyo 105-8461 (JP); OHASHI Hiroki, Tokyo 105-8461 (JP)
(74) Representative: Boult Wade Tennant LLP
(86) International application number: PCT/JP2024/012186
(87) International publication number: WO 2024/204311

(57) **Abstract**

A rod for intra-spinal fixture that reduces damage at the time of fixing with a screw, has high rigidity and high durability against a deformation load, and significantly reduces a discretization risk of fragments even at the time of breakage, is provided. A fixture rod according to one embodiment of the present invention comprises a core member containing a fiber-reinforced resin, and a coating resin layer that coats the core member. A reinforcing member is provided inside the core member along a longitudinal direction of the core member.

## Description

### Technical Field

### Cross Reference

The present application claims priority based on Japanese Patent Application No. 2023-058577 (filed on March 31, 2023), the contents of which are incorporated herein by reference in their entirety.

The present invention relates to a rod for an intra-spinal fixture (in this specification, referred to as a fixture rod for the sake of convenience) used for a fixture for fixing a spine in which breakage fragments are less likely to be discretized.

### Background Art

In related art, fixture rods using metal have been known as fixtures for fixing a spine.

In addition, as such a fixture rod, for example, Patent Literature 1 discloses a spinal pedicle rod including an internally reinforced polymer core at least partially encased in a polymer coating.

### Citation List

### Patent Literature

Patent Literature 1: JP 2011-508623 A

### Summary of Invention

### Technical Problem

A fixture rod using metal is generally excellent in terms of fixing force and strength but has a problem that a magnetic field is affected by magnetization of the metal in the magnetic field at the time of imaging by MRI or the like, image disturbance occurs, and diagnosis based on a captured image is difficult. On the other hand, with the rod disclosed in Patent Literature 1, although there is no such problem, it has been found that it is difficult to impart desired rigidity because the fiber density is lowered, and not only it is less likely to obtain strength and durability, but also there is a problem in safety because spinate fibers are exposed at the time of breakage.

One object of the present invention is to provide a rod for an intra-spinal fixture that reduces damage at the time of fixing with a screw, has high rigidity and high durability against a deformation load, and significantly reduces a discretization risk of fragments even at the time of breakage. Other objects of the present invention will become apparent upon reference to the entirety of the present specification.

### Solution to Problem

A fixture rod according to one embodiment of the present invention comprises a core member containing a fiber-reinforced resin, and a coating resin layer that coats the core member. A reinforcing member is provided inside the core member along a longitudinal direction of the core member.

In the fixture rod according to one embodiment of the present invention, the reinforcing member is a high-elongation metal yarn or fiber member. In addition, in the fixture rod according to one embodiment of the present invention, a tensile strength of the high-elongation metal yarn or fiber member is 150 Mpa or more. In addition, in the fixture rod according to one embodiment of the present invention, the fiber member has a higher elongation than a reinforcing fiber of the fiber-reinforced resin.

In the fixture rod according to one embodiment of the present invention, a diameter of the high-elongation metal yarn or fiber member is in a range of 0.1 mm to 1.0 mm.

In the fixture rod according to one embodiment of the present invention, the high-elongation metal yarn or fiber member is provided in a virtual circle having a diameter of half a diameter of the fixture rod with a center point of the core member as a center as viewed in a section perpendicular to the longitudinal direction of the core member.

In the fixture rod according to one embodiment of the present invention, the core member is formed by laminating a plurality of layers as viewed in a section perpendicular to the longitudinal direction of the core member.

In the fixture rod according to one embodiment of the present invention, each of the plurality of layers of the core member contains a resin, and the reinforcing member is embedded so as to be surrounded by a resin having a thickness in a range of two to three times a diameter of the reinforcing member as viewed in a section perpendicular to the longitudinal direction of the core member.

In the fixture rod according to one embodiment of the present invention, as viewed in a section, the plurality of layers of the core member are formed by alternately forming a plurality of reinforcing fiber layers, and any of a plurality of resin layers, a plurality of reinforcing fiber layers different from fibers of the reinforcing fiber layers, and a plurality of reinforcing fiber layers having fibers obliquely oriented with respect to fiber directions of the reinforcing fiber layers, and the reinforcing member is embedded in one or more layers of the plurality of layers.

In the fixture rod according to one embodiment of the present invention, the reinforcing fiber layers are fiber-reinforced resins, carbon, glass, boron, SiC, or aramid is used as a fiber, and epoxy, phenol, unsaturated polyester, PA, PC, PPSU, POM, PP, PE, ABS, PS, PAEK, or PEEK is used as a resin.

In the fixture rod according to one embodiment of the present invention, a resin of the resin layer is epoxy, phenol, unsaturated polyester, PA, PC, PPSU, POM, PP, PE, ABS, PS, PAEK, or PEEK.

In the fixture rod according to one embodiment of the present invention, the reinforcing fiber layers having the fibers different from the fibers of the reinforcing fiber layer are fiber-reinforced resins, carbon, glass, boron, SiC, or aramid is used as a fiber, and epoxy, phenol, unsaturated polyester, PA, PC, PPSU, POM, PP, PE, ABS, PS, PAEK, or PEEK is used as a resin.

In the fixture rod according to one embodiment of the present invention, in the reinforcing fiber layers or the reinforcing fiber layers having the fibers different from the fibers of the reinforcing fiber layer, the fibers are aligned in one direction, formed in a woven fabric form, or randomly oriented. In addition, in the fixture rod according to one embodiment of the present invention, fiber directions of the reinforcing fiber layers having the fibers obliquely oriented with respect to the fiber direction of the reinforcing fiber layers are obliquely oriented in a range of 10° to 90°.

In the fixture rod according to one embodiment of the present invention, in a case where any of a plurality of resin layers, a plurality of reinforcing fiber layers having fibers different from the fibers of the reinforcing fiber layers, and a plurality of reinforcing fiber layers having fibers obliquely oriented with respect to fiber directions of the reinforcing fiber layers are provided, the layers are the same layers or different layers.

In the fixture rod according to one embodiment of the present invention, the fibers of the reinforcing fiber layer are long fibers. In the fixture rod according to one embodiment of the present invention, a fiber content of the reinforcing fiber layer is 60 weight% or more. In addition, in the fixture rod according to one embodiment of the present invention, fiber directions of the reinforcing fiber layers are aligned, and thicknesses of the layers are in a range of 0.02 mm to 0.3 mm.

In the fixture rod according to one embodiment of the present invention, a marker is provided in the core member.

In the fixture rod according to one embodiment of the present invention, an elongation of the reinforcing member is larger than an elongation of a fiber of the core member.

A fixture rod according to one embodiment of the present invention comprises a core member containing a fiber-reinforced resin. A reinforcing member is provided inside the core member along a longitudinal direction of the core member.

### Advantageous Effects of Invention

According to each of the above-described embodiments of the present invention, it is possible to provide a fixture rod that reduces damage at the time of fixing with a screw, has high rigidity and high durability against a deformation load, and significantly reduces a discretization risk of fragments even at the time of breakage.

### Brief Description of Drawings

Fig. 1 is a diagram illustrating a spinal fixture 10 comprising a fixture rod according to one embodiment of the present invention.
Fig. 2 is a diagram schematically illustrating a section of the fixture rod according to one embodiment of the present invention taken along a plane perpendicular to a central axis of the fixture rod.
Fig. 3 is a diagram schematically illustrating a section of a core member of the fixture rod according to one embodiment of the present invention taken along the plane perpendicular to the central axis of the fixture rod.
Fig. 4 is a diagram schematically illustrating a section of the core member of the fixture rod according to one embodiment of the present invention taken along the plane perpendicular to the central axis of the fixture rod.
Fig. 5 is a diagram schematically illustrating a section of the core member of the fixture rod according to one embodiment of the present invention taken along the plane perpendicular to the central axis of the fixture rod.
Fig. 6 is a diagram schematically illustrating a section of the core member of the fixture rod according to one embodiment of the present invention taken along the plane perpendicular to the central axis of the fixture rod.

### Description of Embodiments

Hereinafter, an embodiment of a fixture rod according to the present invention will be specifically described with reference to the accompanying drawings. Constituent elements common in a plurality of drawings are assigned with the same reference signs throughout the plurality of drawings. It should be noted that each drawing is not necessarily drawn to an accurate scale for convenience of description.

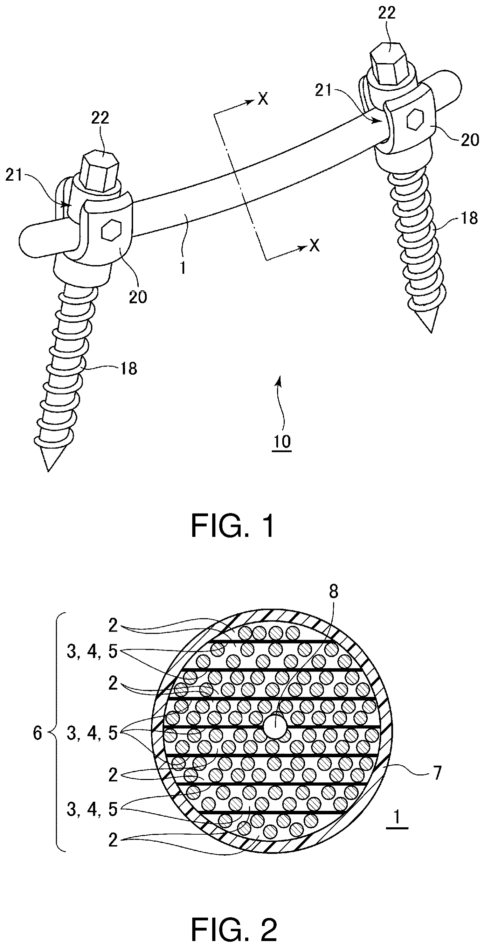

Fig. 1 is a diagram illustrating a spinal fixture 10 comprising a fixture rod 1 according to one embodiment of the present invention. As illustrated in the drawing, the spinal fixture 10 comprises: a plurality of screw members 18 (two screw members 18 in the example illustrated in the drawing) to be fixed to the bone of the spine; a plurality of rod fixing members 20 (two rod fixing members 20 in the example illustrated in the drawing) to be attached to the screw members 18, comprising recesses 21 for receiving the fixture rods and pressing members 22; and the fixture rod 1 to be inserted into the recesses 21 of the plurality of rod fixing members 20 and fixed by the pressing members 22.

Next, the fixture rod 1 according to one embodiment of the present invention used for the spinal fixture 10 will be described with reference to Fig. 2. Fig. 2 illustrates the fixture rod 1 illustrated in Fig. 1 as viewed in X-X section illustrated in the same drawing. As illustrated in the drawing, the fixture rod 1 according to one embodiment of the present invention comprises a core member 6 containing a fiber-reinforced resin and a coating resin layer 7 coating the core member 6, and a reinforcing member 8 is provided inside the core member 6 along a longitudinal direction of the core member 6. In addition, the fixture rod 1 according to one embodiment of the present invention may comprise the core member 6 containing the fiber-reinforced resin, and may be configured such that the reinforcing member 8 is provided inside the core member 6 along the longitudinal direction of the core member 6 and the coating resin layer 7 coating the core member 6 is not provided.

In accordance with the fixture rod according to one embodiment of the present invention, it is possible to provide a fixture rod that reduces damage at the time of fixing with a screw, has high rigidity and high durability against a deformation load, and significantly reduces a discretization risk of fragments even at the time of breakage.

In the fixture rod 1 according to one embodiment of the present invention, the reinforcing member 8 is a high-elongation metal yarn or fiber member. Here, the metal yarn is, for example, a titanium alloy, SUS304, tantalum, or niobium. In addition, the fiber member is, for example, a glass fiber, a nylon fiber, a polyimide fiber, or a PEEK fiber, and has an elongation of 5% or more, desirably 10% or more. In accordance with the fixture rod according to one embodiment of the present invention, it is possible to provide a fixture rod that reduces damage at the time of fixing with a screw, has high rigidity and high durability against a deformation load, and significantly reduces a discretization risk of fragments even at the time of breakage. More specifically, since an elongation of the reinforcing member is larger than an elongation of a fiber of the core member, the reinforcing member is relatively less likely to be broken even in a case where the core member is broken, and the discretization of the fragments is suppressed.

In addition, in the fixture rod 1 according to one embodiment of the present invention, a tensile strength of the high-elongation metal yarn or fiber member is 150 Mpa or more. In this way, it is possible to make the breakage of the reinforcing member less likely to occur due to the impact at the time of breakage of the core member. In addition, in the fixture rod according to one embodiment of the present invention, the fiber member has a higher elongation than that of the reinforcing fiber of the fiber-reinforced resin contained in the core member 6. In this way, it is possible to make the breakage of the reinforcing member less likely to occur due to the impact at the time of breakage of the core member. In addition, in the fixture rod according to one embodiment of the present invention, the reinforcing member has an elongation larger than the elongation of the fiber of the core member. In this way, the reinforcing member is less likely to be broken even at the time of breakage of the core member, and it is possible to suppress the discretization of the fragments.

In the fixture rod 1 according to one embodiment of the present invention, a diameter of the high-elongation metal yarn or fiber member is in a range of 0.1 mm to 1.0 mm. It has been found that, in a case where the diameter is smaller than 0.1 mm, the strength for tethering the core members is insufficient, and on the other hand, in a case where the diameter is larger than 1.0 mm, not only disturbance occurs in MRI imaging, but also weight increase and fiber disturbance easily occur. Thus, it has been found that it is possible to significantly reduce the discretization risk of the fragments even at the time of breakage by being in the above numerical range.

In the fixture rod 1 according to one embodiment of the present invention, the high-elongation metal yarn or fiber member is provided in a virtual circle (X) having a diameter (D/2) that is half of a diameter (D) of the fixture rod, a diameter (D/2) with a center point (O) of the core member 6 as a center in a section perpendicular to the longitudinal direction of the core member 6. In this way, the reinforcing member (the high-elongation metal yarn or fiber member) is provided in the virtual circle, which is a portion where the deformation of the core member due to bending is smaller, and thus, a stress generated in the reinforcing member is reduced. As a result, a load on the reinforcing member itself can be reduced. Accordingly, it is possible to significantly reduce the discretization risk of the fragments even at the time of breakage.

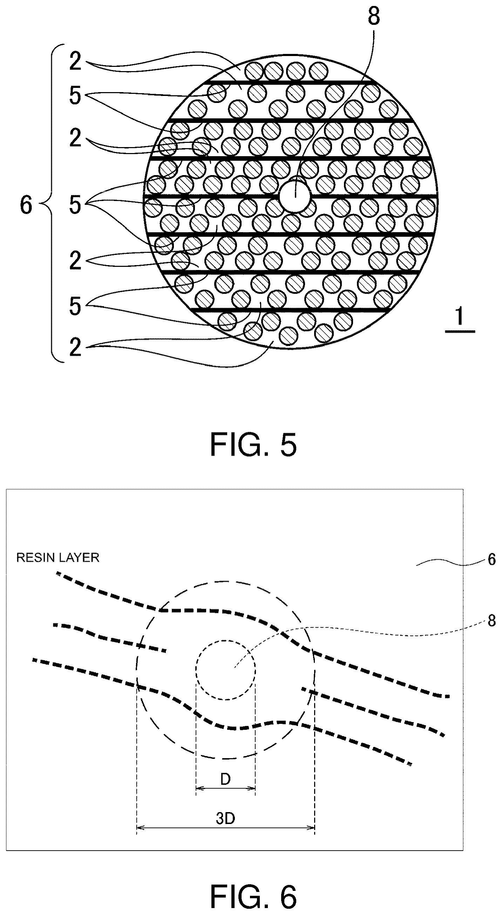

In the fixture rod 1 according to one embodiment of the present invention, the core member 6 is formed by laminating a plurality of layers as viewed in a section perpendicular to the longitudinal direction of the core member 6 (details of laminating the plurality of layers will be described later). In addition, Fig. 6 illustrates an image of a part of a section obtained by cutting the core member of the fixture rod according to one embodiment of the present invention along a plane perpendicular to a central axis of the core member. In the fixture rod 1 according to one embodiment of the present invention, each of the plurality of layers of the core member 6 contains resin, and the reinforcing member 8 is embedded so as to be surrounded by resin having a thickness in a range of two to three times a diameter of the reinforcing member 8 as viewed in the section perpendicular to the longitudinal direction of the core member 6. In this way, in a case where a large stress is generated near the reinforcing member, the fracture easily progresses along the resin or resin layer (for example, a layer of resin illustrated in Fig. 6) surrounding the periphery of the reinforcing member, and as a result, the stress applied to the reinforcing member is relaxed. Accordingly, it is possible to suppress the breakage of the reinforcing member.

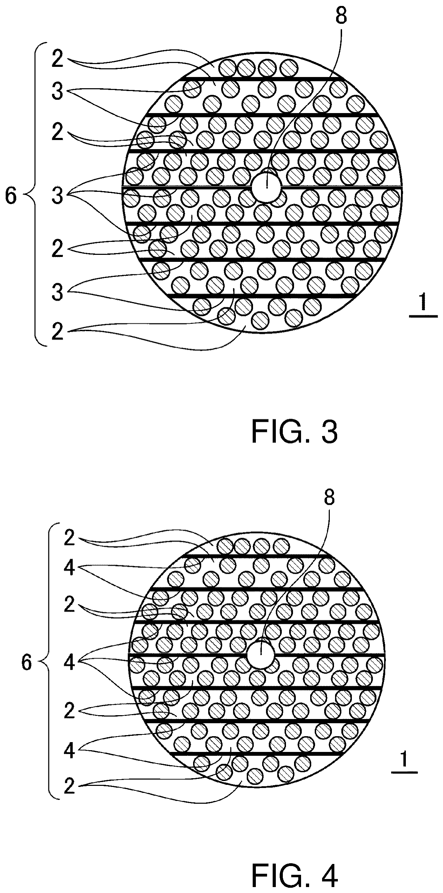

Next, a layer structure of the plurality of layers of the core member 6 of the fixture rod 1 according to one embodiment of the present invention used for the spinal fixture 10 will be described with reference to Figs. 3, 4, and 5. Figs. 3, 4, and 5 illustrate the core member 6 of the fixture rod 1 illustrated in Fig. 1 as viewed in a section taken along line X-X illustrated in Fig. 1. Note that, for the sake of convenience in description, the above-described coating resin layer 7 is omitted.

As illustrated in the drawing, in the plurality of layers of the core member 6 of the fixture rod 1 according to one embodiment of the present invention, as viewed in a section, a plurality of reinforcing fiber layers 2 (in the examples of Figs. 3 to 5, eight reinforcing fiber layers), and any of a plurality of resin layers 3 (in the example illustrated in Fig. 3, seven resin layers 3), a plurality of reinforcing fiber layers 4 (in the example illustrated in Fig. 4, seven reinforcing fiber layers 4) having fibers different from fibers of the reinforcing fiber layers 2, or a plurality of reinforcing fiber layers 5 (in the example illustrated in Fig. 5, seven reinforcing fiber layers 5) having fibers obliquely oriented with respect to fiber directions of the reinforcing fiber layers 2 are alternately formed, and the reinforcing member 8 is embedded in any one layer or in any two or more layers (over two or more layers) of the plurality of layers. Here, the reinforcing fiber layers 2 and any layer of the resin layers 3 (example illustrated in Fig. 3), the reinforcing fiber layers 4 having fibers different from the fibers of the reinforcing fiber layers (example illustrated in Fig. 4), and the reinforcing fiber layers 5 having the fibers obliquely oriented with respect to the fiber directions of the reinforcing fiber layers (example illustrated in Fig. 5) are formed to have a thickness of 0.01 mm to 0.25 mm. However, the thicknesses of the layers may vary depending on the location in the layer (that is, the layers may be formed such that there may be portions with small thicknesses and portions with large thicknesses). In addition, the layers may be formed intermittently (that is, not only there may be portions with small thicknesses as well as portions with large thicknesses, but also portions where the thickness of the layer is 0 may be intercalated).

With the core member 6 of the fixture rod 1 according to one embodiment of the present invention, it is possible to provide a fixture rod that reduces damage at the time of fixing with a screw, has high rigidity and high durability against a deformation load, and significantly reduces the discretization risk of the fragments even at the time of breakage. More specifically, since an elongation of the reinforcing member is larger than an elongation of a fiber of the core member, the reinforcing member is relatively less likely to be broken even in a case where the core member is broken, and the discretization of the fragments is suppressed. In addition, it has been found that, by the layer structure of the fixture rod 1 according to one embodiment of the present invention, at the time of breakage, the stress is concentrated on any of the resin layer, the reinforcing fiber layer having fibers different from those of the reinforcing fiber layer, and the reinforcing fiber layer having fibers obliquely oriented with respect to the fiber direction of the reinforcing fiber layer, which are sandwiched between the reinforcing fiber layers, and thus, the chance of the reinforcing fiber layer becoming spinate when the rod is broken is reduced. As a result, it is possible to significantly enhance the safety to the human body.

In the core member 6 of the fixture rod 1 according to one embodiment of the present invention, the reinforcing fiber layer 2 is a fiber-reinforced resin, in which carbon, glass, boron, SiC, or aramid is used as fibers, and epoxy, phenol, unsaturated polyester, PA, PC, PPSU, POM, PP, PE, ABS, PS, PAEK, or PEEK is used as a resin. In this way, it is possible to increase the bending rigidity and the strength of the fixture rod 1.

In the core member 6 of the fixture rod 1 according to one embodiment of the present invention, the resin of the resin layer 3 is epoxy, phenol, unsaturated polyester, PA, PC, PPSU, POM, PP, PE, ABS, PS, PAEK, or PEEK.

In the core member 6 of the fixture rod according to one embodiment of the present invention, the reinforcing fiber layer 4 having fibers different from the fibers of the reinforcing fiber layer 2 is a fiber-reinforced resin, in which carbon, glass, boron, SiC, or aramid is used as fibers, and epoxy, phenol, unsaturated polyester, PA, PC, PPSU, POM, PP, PE, ABS, PS, PAEK, or PEEK is used as a resin. In this way, it is possible to increase the bending rigidity and the strength of the fixture rod. In addition, the reason for using a reinforcing fiber layer having fibers different from the fibers of the reinforcing fiber layer 2 is that different characteristics such as flexibility and vibration absorbing properties other than rigidity and strength can be imparted by using different materials.

In the core member 6 of the fixture rod according to one embodiment of the present invention, in the reinforcing fiber layer 2 or the reinforcing fiber layer 4 having fibers different from the fibers of the reinforcing fiber layer 2, the fibers may be aligned in one direction, formed in a woven fabric form, or randomly oriented. In addition, in the core member 6 of the fixture rod 1 according to one embodiment of the present invention, the fiber directions of the reinforcing fiber layers 5 having fibers obliquely oriented with respect to the fiber directions of the reinforcing fiber layers 2 may be obliquely oriented in the range of 10° to 90°. In this way, it is possible to realize intended strength and rigidity by arranging an appropriate laminated configuration.

In the core member 6 of the fixture rod 1 according to one embodiment of the present invention, in a case where a plurality of layers of any of the resin layers 3, the reinforcing fiber layers 4 having fibers different from the fibers of the reinforcing fiber layers 2, and the reinforcing fiber layers 5 having fibers obliquely oriented with respect to the fiber directions of the reinforcing fiber layers 2 are provided, the layers are the same layers or different layers (hereinafter, the same applies). In this regard, for example, in a case where two layers are provided, two layers of the resin layer 3 may be provided (in this case, each layer is the same), or two different layers such as the resin layer 3 and the reinforcing fiber layer 4 having fibers different from those of the reinforcing fiber layer, may be provided. In the case of providing three or more layers, a desired combination may be selected and provided from among the above-described layers. In this way, intended strength and rigidity can be realized by arranging an appropriate laminated configuration.

In the core member 6 of the fixture rod 1 according to one embodiment of the present invention, the fibers of the reinforcing fiber layer 2 are long fibers. In this way, the fibers of the reinforcing fiber layer 2 are long fibers, and thus, it is possible to further increase the bending rigidity and the strength.

In addition, in the core member 6 of the fixture rod 1 according to one embodiment of the present invention, it is configured such that a fiber content of the reinforcing fiber layer 2 is 60 weight% or more. In this way, it is possible to form a fixture rod 1 having high rigidity and excellent durability due to fiber layers in which long fibers are focused at high density.

In the core member 6 of the fixture rod 1 according to one embodiment of the present invention, the fiber directions of the reinforcing fiber layers 2 are aligned, and thicknesses of the reinforcing fiber layers 2 are, for example, in a range of 0.02 mm to 0.3 mm. In this way, the density of the resin is made uniform, and it is possible to reduce the variation in strength depending on sites.

Next, a method for manufacturing the fixture rod 1 according to one embodiment of the present invention will be described. First, as step 1, any layer of the reinforcing fiber layers 2, the resin layers 3, the reinforcing fiber layers 4 having the fibers different from the fibers of the reinforcing fiber layers, and the reinforcing fiber layers 5 having fibers obliquely oriented with respect to the fiber directions of the reinforcing fiber layers is cut to predetermined dimensions (layer cutting step). Then, as step 2, a high-elongation metal yarn or fiber member to be used as the reinforcing member 8 is prepared (reinforcing member selecting step). Next, as step 3, the layers are laminated in a predetermined arrangement, and the reinforcing member is mounted between two adjacent layers in consideration of a predetermined position (layer laminating and reinforcing member mounting step). Then, as step 4, the reinforcing member is set in a predetermined mold and molded under predetermined conditions (for example, a temperature is 380°C, and a pressure is 5 to 15 Mpa) (molding step). Next, as step 5, the final shape and dimensions are processed to form the core member 6 (processing step). Finally, as step 6, the coating resin layer 7 is formed on an outer surface of the formed core member 6 by welding a resin tube. In this way, the fixture rod 1 according to one embodiment of the present invention is formed.

With the fixture rod 1 according to one embodiment of the present invention formed in this manner, it is possible to provide a fixture rod that reduces damage at the time of fixing with a screw, has high rigidity and high durability against a deformation load, and significantly reduces the discretization risk of the fragments even at the time of breakage. More specifically, since an elongation of the reinforcing member is larger than an elongation of a fiber of the core member, the reinforcing member is relatively less likely to be broken even in a case where the core member is broken, and the discretization of the fragments is suppressed.

Next, the fixture rod 1 according to one embodiment of the present invention used for the spinal fixture 10 will be described with reference to Fig. 2 again. Fig. 2 illustrates the fixture rod 1 illustrated in Fig. 1 as viewed in X-X section illustrated in the same drawing. The fixture rod 1 according to one embodiment of the present invention comprises the core member 6 containing the fiber-reinforced resin to be described in detail later, the coating resin layer 7 coating the core member 6, and the reinforcing member 8 embedded in the core member 6. A tensile strength of the coating resin layer 7 is 50 Mpa or more and an elongation of 30% or more, and has a thickness in a range of 0.3 mm to 0.4 mm. Such a coating resin layer 7 is provided, and thus, even in a case where the core member 6 inside is damaged. As a result, since it is possible to secure a state where the core member 6 is covered without damaging the coating resin layer 7, it is possible to prevent the exposure of the fibers of the core member 6 to an outside, and it is possible to significantly reduce a damage risk to the human body even at the time of breakage. More particularly, since the elongation of the coating resin is sufficiently large relative to a standard elongation of about 2% of carbon fibers, the breakage occurs from the carbon fibers. In addition, when the coating resin has a certain thickness and strength or more, the breakage of the coating resin can be suppressed against an impact force accompanying the breakage of the carbon fiber. However, when the thickness of the coating resin is in a range exceeding 0.4 mm, breakage of the coating hardly occurs, but an outer diameter itself of the rod increases, and the influence on indwelling in the body increases. In addition, due to the core member to be described later, damage at the time of fixing the screw can be reduced, high rigidity can be achieved, and a durability against a deformation load can be improved. However, the coating resin layer further exhibits the above technical effects. Here, in the fixture rod 1 according to one embodiment of the present invention, a marker is embedded in the core member 6. Note that, the marker is made of a radiopaque substance and plays a role of indicating a position of an implant product in the body.

In the fixture rod 1 according to one embodiment of the present invention, a fiber volume content (VF) in the vicinity of a surface layer of the core member 6 is preferably in a range of 50% to 70%, and more preferably in a range of 55% to 65%. Here, the vicinity of the surface layer of the core member 6 refers to a region 0.2 mm inward from a surface of the core member in a center direction. In a case where the fiber volume content (VF) in the vicinity of the surface layer of the core member 6 is 50% or more, the bending rigidity can be efficiently increased, and a rod less likely to be damaged can be formed. In addition, in a case where a volume ratio in the vicinity of the surface of the core member is 50% or more, the bonding to the coating resin is not completely integrated. That is, while the resin on the surface of the core member and the coating resin are easily firmly integrated, the carbon fibers on the surface of the core member and the coating resin are relatively weakly bonded. In contrast, when the fiber volume content (VF) in the vicinity of the surface layer of the core member 6 exceeds 70%, since the amount of resin on the surface is too small, sufficient bonding cannot be obtained between the coating resin and the resin of the core member. Since the core member and the coating resin are not completely integrated but are weakly bonded to each other to a certain extent or more, when the core member inside is broken, a crack develops between the surface of the core member and the coating resin to release stress. As a result, the breakage of the coating resin is less likely to occur.

In the fixture rod 1 according to one embodiment of the present invention, the resin of the core member 6 and the resin of the coating resin layer 7 are the same thermoplastic resin. Examples of such a thermoplastic resin include PEEK, nylon, PPSU, and PET. In this way, the core member and the coating resin can be stably integrated.

In the fixture rod according to one embodiment of the present invention, the resin of the coating resin layer is formed by welding a resin tube. In this way, the coating layer having a stable thickness and quality can be formed by molding the coating layer into a tubular shape in advance.

The dimensions, materials, and arrangement of components described in the present specification are not limited to those explicitly described in the embodiment. The components can have any modified dimensions, materials, and arrangement that are included within the scope of the present invention. In addition, components not explicitly described in the present specification can also be added to the described embodiments, or some of the components described in the embodiments can be omitted.

### Reference Signs List

1 Fixture rod
2 Reinforcing fiber layer
3 Resin layer
4 Reinforcing fiber layers having fibers different from fibers of reinforcing fiber layers 2
5 Reinforcing fiber layers having fibers obliquely oriented with respect to fiber directions of reinforcing fiber layers 2
6 Core member
7 Coating resin layer
8 Reinforcing member
10 Spinal fixture
18 Screw member
20 Rod fixing member
21 Recess
22 Pressing member

## Claims

1. A fixture rod comprising:
a core member containing a fiber-reinforced resin; and
a coating resin layer that coats the core member,
wherein a reinforcing member is provided inside the core member along a longitudinal direction of the core member.

2. The fixture rod according to claim 1, wherein the reinforcing member is a high-elongation metal yarn or fiber member.

3. The fixture rod according to claim 2, wherein a tensile strength of the high-elongation metal yarn or fiber member is 150 Mpa or more.

4. The fixture rod according to claim 2, wherein the fiber member has a higher elongation than a reinforcing fiber of the fiber-reinforced resin.

5. The fixture rod according to claim 2, wherein a diameter of the high-elongation metal yarn or fiber member is in a range of 0.1 mm to 1.0 mm.

6. The fixture rod according to claim 2, wherein the high-elongation metal yarn or fiber member is provided in a virtual circle having a diameter of half a diameter of the fixture rod with a center point of the core member as a center as viewed in a section perpendicular to the longitudinal direction of the core member.

7. The fixture rod according to claim 1, wherein the core member is formed by laminating a plurality of layers as viewed in a section perpendicular to the longitudinal direction of the core member.

8. The fixture rod according to claim 1, wherein each of the plurality of layers of the core member contains a resin, and the reinforcing member is embedded so as to be surrounded by a resin having a thickness in a range of two to three times a diameter of the reinforcing member as viewed in a section perpendicular to the longitudinal direction of the core member.

9. The fixture rod according to claim 7, wherein, as viewed in a section, the plurality of layers of the core member are formed by alternately forming a plurality of reinforcing fiber layers, and any of a plurality of resin layers, a plurality of reinforcing fiber layers different from fibers of the reinforcing fiber layers, and a plurality of reinforcing fiber layers having fibers obliquely oriented with respect to fiber directions of the reinforcing fiber layers, and the reinforcing member is embedded in one or more layers of the plurality of layers.

10. The fixture rod according to claim 9, wherein the reinforcing fiber layers are fiber-reinforced resins, carbon, glass, boron, SiC, or aramid is used as a fiber, and epoxy, phenol, unsaturated polyester, PA, PC, PPSU, POM, PP, PE, ABS, PS, PAEK, or PEEK is used as a resin.

11. The fixture rod according to claim 9, wherein a resin of the resin layer is epoxy, phenol, unsaturated polyester, PA, PC, PPSU, POM, PP, PE, ABS, PS, PAEK, or PEEK.

12. The fixture rod according to claim 9, wherein the reinforcing fiber layers having the fibers different from the fibers of the reinforcing fiber layer are fiber-reinforced resins, carbon, glass, boron, SiC, or aramid is used as a fiber, and epoxy, phenol, unsaturated polyester, PA, PC, PPSU, POM, PP, PE, ABS, PS, PAEK, or PEEK is used as a resin.

13. The fixture rod according to claim 9, wherein, in the reinforcing fiber layers or the reinforcing fiber layers having the fibers different from the fibers of the reinforcing fiber layer, the fibers are aligned in one direction, formed in a woven fabric form, or randomly oriented.

14. The fixture rod according to claim 9, wherein fiber directions of the reinforcing fiber layers having the fibers obliquely oriented with respect to the fiber direction of the reinforcing fiber layers are obliquely oriented in a range of 10° to 90°.

15. The fixture rod according to claim 9, wherein, in a case where any of a plurality of resin layers, a plurality of reinforcing fiber layers having fibers different from the fibers of the reinforcing fiber layers, and a plurality of reinforcing fiber layers having fibers obliquely oriented with respect to fiber directions of the reinforcing fiber layers are provided, the layers are the same layers or different layers.

16. The fixture rod according to claim 9, wherein the fibers of the reinforcing fiber layer are long fibers.

17. The fixture rod according to claim 9, wherein a fiber content of the reinforcing fiber layer is 50 vol% or more.

18. The fixture rod according to claim 9, wherein fiber directions of the reinforcing fiber layers are aligned, and thicknesses of the reinforcing fiber layers are in a range of 0.02 mm to 0.3 mm.

19. The fixture rod according to claim 1, wherein a marker is provided in the reinforcing member.

20. The fixture rod according to claim 1, wherein an elongation of the reinforcing member is larger than an elongation of a fiber of the core member.

21. A fixture rod comprising:
a core member containing a fiber-reinforced resin,
wherein a reinforcing member is provided inside the core member along a longitudinal direction of the core member.
